# EUROPEAN PATENT SPECIFICATION

(11) **EP 4 208 095 B1**
(45) Date of publication and mention of the grant of the patent: **01.10.2025**
(21) Application number: 21759019.9
(22) Date of filing: 19.08.2021
(51) Int. Cl.: A61B 5/291, A61B 5/296, A61B 5/369, A61B 5/389

(54) **SOFT AND DRY ELECTRODE**
WEICHE UND TROCKENE ELEKTRODE
ÉLECTRODE SOUPLE ET SÈCHE

(30) Priority: 03.09.2020 CH 10872020
(43) Date of publication of application: 12.07.2023
(73) Proprietor: Dätwyler Schweiz AG, 6467 Schattdorf (CH)
(72) Inventor: VRIJENS, Ronny, 3721 Vliermaalroot (BE); LUCCHINI, Mattia Alberto, 6003 Luzern (CH); KAELIN, Raphael, 6052 Hergiswil (CH); SEGESSENMANN, Michael, 6463 Bürglen (CH)
(74) Representative: Prins Intellectual Property AG
(86) International application number: PCT/CH2021/050019
(87) International publication number: WO 2022/047595

(56) References cited:
- JP-A- 2019 097 733

## Description

### Technical Field

The invention relates to soft and dry electrodes for detection of bioelectric signals in applications such as electroencephalography (EEG), electrocardiography (ECG) or electromyography (EMG).

### Prior Art

Commercially available 'dry' EEG headsets are often equipped with metal dry electrodes, which causes subjects to feel pain after wearing the headsets for a while. A possible solution is combining such electrodes with a spring-like system to avoid high skin pressure.

Yet another approach is the use of soft polymer-based dry electrodes. By mixing the elastic polymer with additives the conductivity can be improved while maintaining the required elasticity for high user comfort. The polymer based dry electrodes can have a comb shape design (fingers or legs) to improve skin contact on hairy skin (e.g. on the scalp). Such fingers or legs can have at least a partial coating on a surface of the electrode in contact with the skin in order to lower the skin impedance and provide an improved signal quality.

Thus, soft and dry electrodes are increasingly used for long term biopotential measurements such as EEG and ECG. Next to being soft, the additional fact that such electrodes can be applied without the use of a conductive gel provides the measurement procedure with considerable benefits such as a decreased risk of skin irritation and the avoidance of a decrease of signal quality due to gel drying.

Examples of such soft and dry electrodes are described by Chen et al. in "Polymer-based dry electrodes for high user comfort ECG/EEG measurements" (Chen, Yun-Hsuan; Op de Beeck, Maaike; Carrette, Evelien; Vanderheyden, Luc; Grundlehner, Bernard; Mihajlovic, Vojkan; Boon, Paul; Van Hoof, Chris; Apprimus Verlag; Aachen; 8th International Conference Exhibition on Integration Issues of Miniaturized Systems - MEMS, NEMS, ICs and Electronic Components; 2014; pp. 329 - 336), Chen et al. in "Soft, Comfortable Polymer Dry Electrodes for High Quality ECG and EEG Recording" (Sensors 2014, 14, 23758-23780; doi:10.3390/s141223758) or WO2016080804.

These soft and dry electrodes comprise a base plate and a plurality of pins for contacting an area of interest to be measured. The pins may have a tapered portion and a protruding portion. The electrode tips are made of a flexible or soft matrix material which is provided with an electrically conductive material. The electrodes may have a knob on its upper side of the base plate opposite of the pins for electrically connecting the electrode.

Upon exertion of force on the soft electrode (e.g. by means of a strap, band, headset or head-cap) the legs may move in an uncontrolled manner, not offering the intended brush function to move aside hair and provide for a direct contact between electrode and skin surface.

One possible solution for this problem is a pre-orientation of the legs (as described in EP2827770) so that on applying the electrode to the subject area (e.g. a scalp) these legs are disposed at a non-perpendicular angle to that subject area. A downside of this approach however is the adopted manufacturing process involving a 3D-printing step which is not suitable for up-scaling towards high volume productions.

JP 2019 097733 A relates to an electrode for measuring brain activity. The electrode has a stiff support body and several arms attached at the side of the stiff support body. A ball is formed at the tip of the arms to contact the scalp of a person. The arms are flexible and bend when a force is applied to the electrode. The electrode has a complex shape with several undercuts, which makes it difficult to manufacture in a cost-efficient way.

### Summary of the Invention

It is an objective of the invention to provide a soft electrode for measuring bioelectric signals of an individual avoiding the problems of the prior art and being suitable for high volume production.

At least one of the objectives is achieved by a soft electrode for measuring bioelectric signals of an individual according to claim 1. The soft electrode comprises a support body having a contact side facing the individual when applying the electrode to the individual and a connector side opposite the contact side. The connector side serves for connecting the electrode to an electronic circuit. The support body defines a central axis arranged centrally through the contact side and the connector side. The electrode further comprises a plurality of outer pins located at a radially outer region of the support body for contacting an area of interest to be measured. The plurality of outer pins being supported and arranged on the contact side of the support body. The electrode is made of elastomeric material and has electrically conductive properties. The support body has a dome-like shape having a concave side and a convex side, wherein the concave side forms the contact side of the support body. The support body is designed with a flexibility such that after applying the electrode to the individual a force exerted centrally onto the connector side and parallel to the central axis leads to an upwards bending of the radially outer region of the support body in direction of the connector side. The upwards bending of the radially outer region of the support body leads to a tilting of the plurality of outer contact pins relative to the central axis such that a tip of the outer contact pins moves radially outwards along the area of interest of the individual.

In other words, upon force exertion on the top surface of the support body, the flexible support body starts bending such that the tip of the pins or legs move in an outward direction (i.e. away from the centre of the electrode) in order to 'brush aside' any hair that is hindering direct contact between electrode and bare skin surface of the individual to be measured. Additionally, the use of elastomeric materials, e.g. a thermoset elastomer or a thermoplastic elastomer (TPE) and the dome-like shape of the support body with a plurality of pins parallel to the central axis of the support body allow for production methods such as injection moulding, compressing moulding, injection transfer moulding, injection compression moulding, etc.. These type of production methods allow high volume production of the electrodes.

Further embodiments of the invention are set forth in the dependent claims.

In some embodiments a longitudinal axis of each of the plurality of outer pins is parallel to the central axis.

In some embodiments, the electrode may further comprise a plurality of inner contact pins located at an inner region of the support body closer to the central axis than the outer region of the support body and being dimensioned to contact the individual after the tilting of the outer contact pins occurred.

In some embodiments, the plurality of outer contact pins and the plurality of inner contact pins may have the same length. Alternatively, the plurality of outer contact pins and the plurality of inner contact pins may have different length, preferably the inner contact pins have a shorter length than the outer contact pins. For instance, the pins positioned in a more central region of the electrode can have a shorter length so that they will only start touching the skin surface (e.g. scalp) when the outer pins have been tilting outwards and moving aside hair in order to prepare a bare skin for these central pins to touch.

In some embodiments, the plurality of outer contact pins and the plurality of inner contact pins may be arranged and dimensioned such that while applying the electrode to the individual the plurality of outer contact pins contact the area of interest before the plurality of inner contact pins.

In some embodiments, the plurality of outer contact pins and/or the plurality of inner contact pins may comprise a cone-shaped base portion and a cylinder-shaped free end portion. The free end portion forms the tip of the pin.

In some embodiments, the support body may be a dome-shaped disc, preferably a circular disc. The disc in the sense of the invention may have a quasi-circular shape such as oval, or polygonal, e.g. triangular, penta- or hexagonal or the like.

In some embodiments, the support body may comprise a central disc, preferably a circular disc with a plurality of legs directed radially outwards, at an angle to the central axis of less than 90 degrees, preferably 30 to 70 degrees, and defining the outer region of the dome-shaped support body, wherein the plurality of outer contact pins are arranged at the free end of the legs.

In some embodiments, the connector side of the support body may be provided with a knob or a so-called male snap fit for electrically connecting the electrode to an electronic circuit. The knob or male snap fit may be of the same soft electrically conductive material as the electrode or in a rigid material (e.g. metal, plastic) for facilitating the connection with an electronic circuit.

In some embodiments, the tip of the plurality of outer contact pins may comprise an inclined surface facing towards the central axis of the support body.

In some embodiments the connector side of the support body may be provided with slits or grooves surrounding a base portion of the contact pins to increase flexibility of the support body.

In some embodiments, the elastomeric material of the electrode may be a thermoset elastomer or a thermoplastic elastomer.

The elastomeric material can be, for example, a synthetic or natural rubber, such as butyl rubber, isoprene rubber, butadiene rubber, halogenated butyl rubber (e.g., bromobutyl rubber), ethylene propylene terpolymer, silicone rubber, fluoro- or perfluoroelastomers, chlorosulfonate, polybutadiene, butyl, neoprene, nitrile, polyisoprene, buna-N, copolymer rubbers such as ethylene-propylene (EPR), ethylene-propylene-diene monomer (EPDM), acrylonitrile-butadiene (NBR or HNBR) and styrene-butadiene (SBR), blends such as ethylene or propylene-EPDM, EPR, or NBR, combinations thereof. The term "synthetic rubbers" also should be understood to encompass materials which alternatively may be classified broadly as thermoplastic or thermosetting elastomers such as polyurethanes, silicones, fluorosilicones, styrene-isoprene-styrene (SIS), and styrene-butadiene-styrene (SBS), as well as other polymers which exhibit rubber-like properties such as plasticized nylons, polyolefins, polyesters, ethylene vinyl acetates, fluoropolymers, and polyvinyl chloride.

Good results may be achieved with ethylene propylene diene mono rubber (EPDM), silicone rubber (SR), liquid silicone rubber (LSR), butyl rubber, isoprene or nitrile rubber.

In some embodiments, the conductive properties of the electrode may be achieved by adding conductive material to the elastomeric material. The conductive material may be carbon black, silver coated glass spheres, silver particles, Ag-coated aluminium beads, Ag-coated glass fibres, graphene, carbon nanotubes, graphite, stainless steel fibres, or any other suitable material. Conductive properties of the electrode may also be achieved by coating the electrode with conductive material, e.g. Ag-AgCl or PEDOT:PSS.

In some embodiments, the coating may also be applied in addition to the conductive elastomeric material. Such additional coating may be applied on the tips of the pins only.

In some embodiments, the electrode may be formed as a single piece.

### Brief Explanation of the Figures

The invention is described in greater detail below with reference to embodiments that are illustrated in the figures. The figures show:
- Fig. 1: a bottom view (a), a side view (b) and a perspective view (c) of an electrode with a dome-shaped circular disk;
- Fig. 2: sectional views of the electrode of Fig. 1 before (a) and after (b) exerting a force to the electrode;
- Fig. 3: a bottom view (a), a side view (b) and a perspective view (c) of an electrode with a dome-like support body with legs; and
- Fig. 4: sectional views of the electrode of Fig. 3 before (a) and after (b) exerting a force to the electrode.

### Embodiments of the Invention

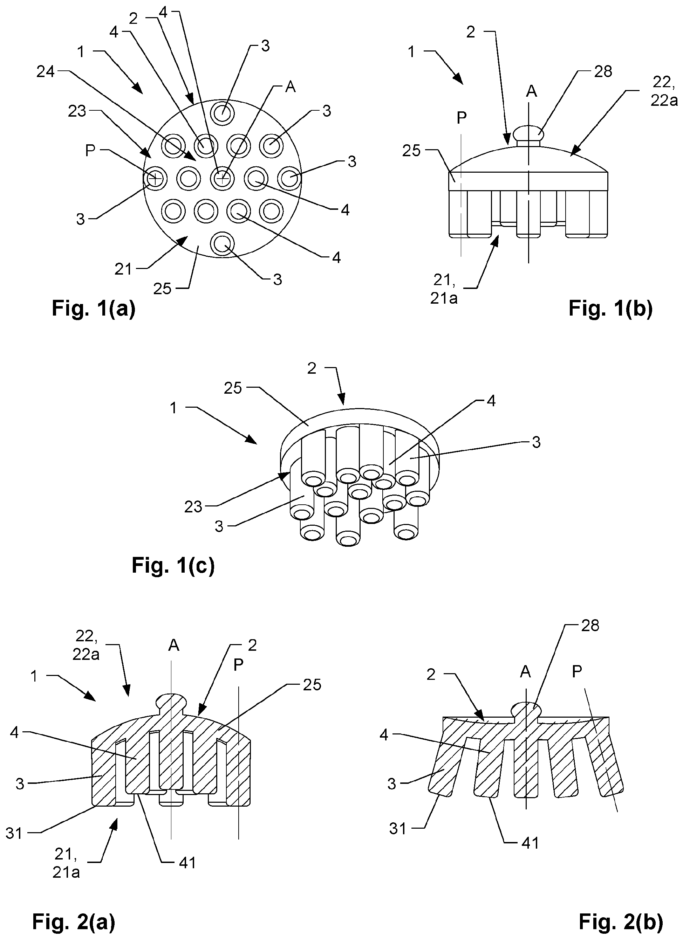

Fig. 1 shows a bottom view (a), a side view (b) and a perspective view (c) of a soft and dry electrode 1 for measuring bioelectric signals of an individual. Fig. 2 shows sectional views of the electrode of Fig. 1 before (a) and after (b) exerting a force to the electrode.

The electrode 1 is made of elastomeric material, which is provided with conductive additives and/or is at least partially coated with a conductive coating. The electrode 1 is formed as a single piece and comprises a dome-shaped support body 2, several outer contact pins 3 and several inner contact pins 4. The support body 2 forms a concave contact side 21 supporting the contact pins 3,4 for contacting the individual and a convex connector side 22 opposite the contact side 21. The connector side 22 is provided with a connector knob 28 for electrically connecting the electrode 1 to an electronic circuit. The support body 2 defines a central axis A arranged centrally through the contact side 21 and the connector side 22.

The support body 2 supports the plurality of outer pins 3, which are arranged at a radially outer circumferential region 23 of the support body 2. A central axis P of each pin 3 is parallel to the central axis A of the support body. In other words, when applying the electrode to the individual, the pins 3 touch the contact area of the individual in a perpendicular direction.

The dome-shaped support body 2 is flexible such that the radially outer region 23 of the support body 2 may bend upwards (i.e. away from the individual) when a force is applied centrally onto the connector side 22 of the electrode 1 and parallel to the central axis A of the support body 2. Thus, while exerting force onto the connector side 22 of the electrode 1, each outer pin 3 starts to tilt and a tip 31 of each outer pin 3 slides in a radially outward direction along the skin of the individual and thereby brushes through hair that may be present. The contact of the electrode to the individual is thereby increased. To increase rigidity of each outer pin 3, it may comprise a cone-shaped base portion and a cylinder-shaped tip portion.

The electrode shown in Fig. 1 and Fig. 2 additionally comprises inner pins 4. The outer and inner pins 3, 4 have the same length such that a tip 41 of the inner pins 4 is offset along the central axis A in direction of the connector side 22. Thus, while applying the electrode 1 to the individual, the outer pins 3 contact the contact area of the individual first.

To facilitate the sliding movement on the skin, the free end of the pins may be rounded or provided with an inclined surface facing towards the central axis of the support body.

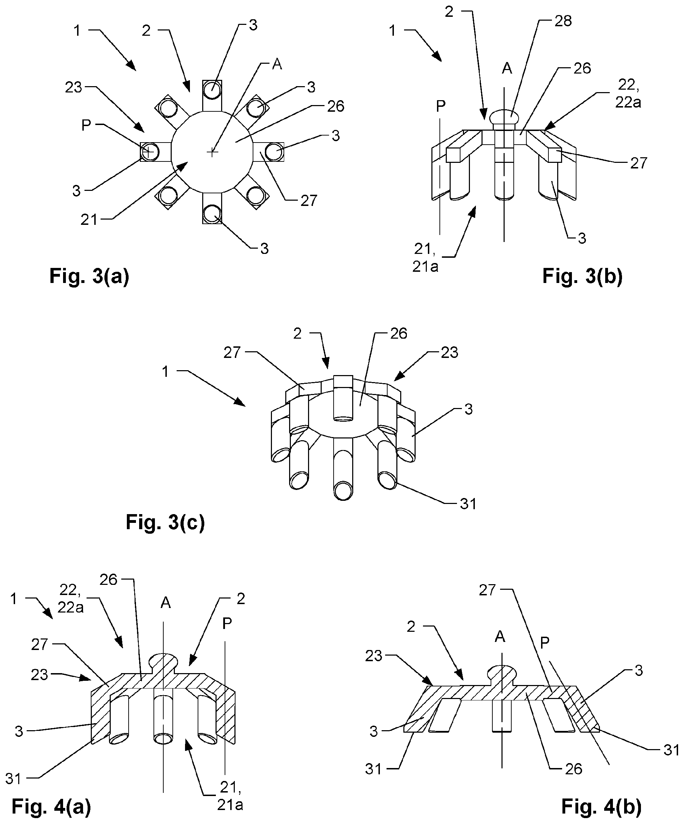

Fig. 3 shows a bottom view (a), a side view (b) and a perspective view (c) of a further embodiment of a soft and dry electrode 1 for measuring bioelectric signals of an individual. Fig. 4 shows sectional views of the electrode of Fig. 3 before (a) and after (b) exerting a force to the electrode.

In contrast to the electrode 1 of Fig. 1 the support body 2 of the electrode 1 of Fig. 3 comprises a central circular disc 26 and a plurality of legs 27. The legs 27 are evenly and circumferentially arranged and directed radially outwards at an angle to the central axis A of less than 90 degrees, preferably 30 to 70 degrees. The legs 27 define the outer region 23 of the dome-shaped support body 2. The plurality of outer contact pins 3 are arranged at the free end of the legs 27. In the embodiment shown, inner pins are not present. Alternatively, inner pins, which touch the individual only after the legs start bending may be present.

While exerting a force to the electrode 1, the outer tips of the legs 27 forming the outer region 23 of the support body 2 move upwards in direction of the connector side 23, i.e. approximately parallel to the central axis A. Thereby, the outer pins 3 tilt and the tip 31 of each outer pin 3 moves outwards, radially away from the central axis A and slides along the skin of the individual.

### Reference Signs

- 1: electrode
- 2: support body
- 21: contact side of support body
- 21a: concave side
- 22: connector side of support body
- 22a: convex side
- 23: outer region of support body
- 24: inner region of support body
- 25: dome-shaped circular disc
- 26: central circular disc
- 27: leg
- 28: knob
- 3: outer contact pin
- 31: tip of outer contact pin
- 4: inner contact pin
- 41: tip of inner contact pin
- A: central axis
- P: pin axis

## Claims

1. Soft electrode (1) for measuring bioelectric signals of an individual, the electrode (1) comprising
a support body (2) having
a contact side (21) facing the individual when applying the electrode (1) to the individual and
a connector side (22) opposite the contact side (21), and
the support body (2) further defining a central axis (A) arranged centrally through the contact side (21) and the connector side (22);
the electrode (1) further comprising a plurality of outer contact pins (3) located at a radially outer region (23) of the support body (2) for contacting an area of interest to be measured,
the plurality of outer contact pins (3) being supported and arranged on the contact side (21) of the support body (2);
wherein the electrode (1) is made of elastomeric material and has conductive properties;
further wherein the support body (2) has a dome-like shape having a concave side (21a) and a convex side (22b), wherein the concave side (21a) forms the contact side (21) of the support body (2), and
wherein the support body (2) is designed with a flexibility such that after applying the electrode to the individual a force exerted centrally onto the connector side (22) and parallel to the central axis (A) leads to an upwards bending of the radially outer region (23) of the support body (2) in direction of the connector side (22),
wherein the upwards bending of the radially outer region (23) of the support body (2) leads to a tilting of the plurality of outer contact pins (3) relative to the central axis (A) such that a tip (31) of the outer contact pins (3) moves radially outwards along the area of interest of the individual.

2. Soft electrode according to claim 1, **characterized in that** a longitudinal axis (P) of each of the plurality of outer contact pins (3) is parallel to the central axis (A).

3. Soft electrode according to claim 1 or 2, **characterized in that** the electrode (1) further comprises a plurality of inner contact pins (4) located at an inner region (24) of the support body (2) closer to the central axis (A) than the outer region (23) of the support body (2) and being dimensioned to contact the individual after the tilting of the outer contact pins (3) occurred.

4. Soft electrode according to claim 3, **characterized in that** the plurality of outer contact pins (3) and the plurality of inner contact pins (4) have the same length.

5. Soft electrode according to claim 3 or 4, **characterized in that** the plurality of outer contact pins (3) and the plurality of inner contact pins (4) are arranged and dimensioned such that while applying the electrode (1) to the individual the plurality of outer contact pins (3) contact the area of interest before the plurality of inner contact pins (4).

6. Soft electrode according to one of the preceding claims, **characterized in that** the plurality of outer contact pins (3) and/or the plurality of inner contact pins (4) comprise a cone-shaped base portion and a cylinder-shaped free end portion.

7. Soft electrode according to one of the preceding claims, **characterized in that** the support body (2) is a dome-shaped disc, preferably a circular disc (25).

8. Soft electrode according to one of the claims 1 to 6, **characterized in that** the support body (2) comprises a central disc, preferably a circular disc (26) with a plurality of legs (27) directed radially outwards, at an angle to the central axis (A) of less than 90 degrees, preferably 30 to 70 degrees, and defining the outer region (23) of the dome-shaped support body (2), wherein the plurality of outer contact pins (3) are arranged at the free end of the legs (27).

9. Soft electrode according to one of the preceding claims, **characterized in that** the connector side (22) of the support body (2) is provided with a knob (28) for electrically connecting the electrode (1) to an electronic circuit.

10. Soft electrode according to one of the preceding claims, **characterized in that** the tip (31) of the plurality of outer contact pins (3) comprises an inclined surface facing towards the central axis (A) of the support body (2).

11. Soft electrode according to one of the preceding claims, **characterized in that** the elastomeric material of the electrode (1) is a thermoset elastomer or a thermoplastic elastomer.

12. Soft electrode according to one of the preceding claims, **characterized in that** the electrode is formed as a single piece.

## Patentansprüche

1. Weiche Elektrode (1) zum Messen bioelektrischer Signale eines Individuums, wobei die Elektrode (1) einen Stützkörper (2) umfasst, der eine Kontaktseite (21) aufweist, die dem Individuum zugewandt ist, wenn die Elektrode (1) an dem Individuum angelegt wird, und eine Anschlussseite (22), die der Kontaktseite (21) gegenüberliegt, und wobei der Stützkörper (2) weiter eine Mittelachse (A) definiert, die mittig durch die Kontaktseite (21) und die Anschlussseite (22) angeordnet ist; die Elektrode (1) umfasst ferner mehrere äussere Kontaktpins (3), die in einem radial äusseren Bereich (23) des Stützkörpers (2) angeordnet sind, um einen zur Messung vorgesehen Bereich zu kontaktieren, wobei die Mehrzahl von äusseren Kontaktpins (3) auf der Kontaktseite (21) des Stützkörpers (2) gelagert und angeordnet ist; wobei die Elektrode (1) aus elastomerem Material besteht und leitfähige Eigenschaften aufweist; wobei der Stützkörper (2) eine kuppelartige Form mit einer konkaven Seite (21a) und einer konvexen Seite (22b) aufweist, wobei die konkave Seite (21a) die Kontaktseite (21) des Stützkörpers (2) bildet, und wobei der Stützkörper (2) derart mit einer Flexibilität ausgebildet ist, dass nach dem Anlegen der Elektrode an dem Individuum eine Kraft, die mittig auf die Anschlussseite (22) und parallel zur Mittelachse (A) ausgeübt wird, zu einer nach oben gerichteten Biegung des radial äusseren Bereichs (23) des Stützkörpers (2) in Richtung der Anschlussseite (22) führt, wobei die Aufwärtsbiegung des radial äusseren Bereichs (23) des Stützkörpers (2) zu einer Kippung der Mehrzahl der äusseren Kontaktpins (3) relativ zur Mittelachse (A) führt, so dass sich eine Spitze (31) der äusseren Kontaktpins (3) radial nach aussen entlang des zur Messung vorgesehenen Bereichs des Individuums bewegt.

2. Weiche Elektrode nach Anspruch 1, **dadurch gekennzeichnet, dass** eine Längsachse (P) von jedem der Mehrzahl der äusseren Kontaktpins (3) parallel zur Mittelachse (A) verläuft.

3. Weiche Elektrode nach Anspruch 1 oder 2, **dadurch gekennzeichnet, dass** die Elektrode (1) ferner mehrere innere Kontaktpins (4) aufweist, die in einem inneren Bereich (24) des Stützkörpers (2) näher an der Mittelachse (A) als der äussere Bereich (23) des Stützkörpers (2) angeordnet sind und so dimensioniert sind, dass sie die Person berühren, nachdem die Kippung der äusseren Kontaktpins (3) aufgetreten ist.

4. Weiche Elektrode nach Anspruch 3, **dadurch gekennzeichnet, dass** die Mehrzahl der äusseren Kontaktpins (3) und die Mehrzahl der inneren Kontaktpins (4) die gleiche Länge aufweisen.

5. Weiche Elektrode nach Anspruch 3 oder 4, **dadurch gekennzeichnet, dass** die Mehrzahl der äusseren Kontaktpins (3) und die Mehrzahl der inneren Kontaktpins (4) derart angeordnet und dimensioniert sind, dass beim Anlegen der Elektrode (1) an das Individuum die Mehrzahl der äusseren Kontaktpins (3) den zur Messung vorgesehenen Bereich vor der Mehrzahl der inneren Kontaktpins (4) berühren.

6. Weiche Elektrode nach einem der vorhergehenden Ansprüche, **dadurch gekennzeichnet, dass** die Mehrzahl der äusseren Kontaktpins (3) und/oder die Mehrzahl der inneren Kontaktpins (4) einen kegelförmigen Basisabschnitt und einen zylinderförmigen freien Endabschnitt umfassen.

7. Weiche Elektrode nach einem der vorhergehenden Ansprüche, **dadurch gekennzeichnet, dass** der Stützkörper (2) eine kuppelförmige Scheibe, vorzugsweise eine kreisförmige Scheibe (25) ist.

8. Weiche Elektrode nach einem der Ansprüche 1 bis 6, **dadurch gekennzeichnet, dass** der Stützkörper (2) eine Mittelscheibe, vorzugsweise eine kreisförmige Scheibe (26), mit mehreren radial nach aussen gerichteten Schenkeln (27) in einem Winkel zur Mittelachse (A) von weniger als 90 Grad, bevorzugt 30 bis 70 Grad, aufweist und den äusseren Bereich (23) des kuppelförmigen Stützkörpers (2) definiert, wobei die Mehrzahl der äusseren Kontaktpins (3) am freien Ende der Schenkel (27) angeordnet sind.

9. Weiche Elektrode nach einem der vorhergehenden Ansprüche, **dadurch gekennzeichnet, dass** die Anschlussseite (22) des Stützkörpers (2) mit einem Knopf (28) zum elektrischen Verbinden der Elektrode (1) mit einer elektronischen Schaltung versehen ist.

10. Weiche Elektrode nach einem der vorhergehenden Ansprüche, **dadurch gekennzeichnet, dass** die Spitze (31) der Mehrzahl der äusseren Kontaktpins (3) eine zur Mittelachse (A) des Stützkörpers (2) geneigte Fläche aufweist.

11. Weiche Elektrode nach einem der vorhergehenden Ansprüche, **dadurch gekennzeichnet, dass** das elastomere Material der Elektrode (1) ein duroplastisches Elastomer oder ein thermoplastisches Elastomer ist.

12. Weiche Elektrode nach einem der vorhergehenden Ansprüche, **dadurch gekennzeichnet, dass** die Elektrode als ein Stück ausgebildet ist.

## Revendications

1. Électrode souple (1) pour mesurer des signaux bioélectriques d'un individu, l'électrode (1) comprenant :
un corps de support (2) ayant
un côté contact (21) faisant face à l'individu lorsqu'on applique l'électrode (1) sur l'individu et
un côté connecteur (22) opposé au côté contact (21), et
le corps de support (2) définissant en outre un axe central (A) agencé de manière centrale à travers le côté contact (21) et le côté connecteur (22) ;
l'électrode (1) comprenant en outre une pluralité de broches de contact extérieures (3) situées sur une région radialement extérieure (23) du corps de support (2) pour être en contact avec une zone d'intérêt à mesurer,
la pluralité de broches de contact extérieures (3) étant supportée et agencée sur le côté contact (21) du corps de support (2) ;
dans laquelle l'électrode (1) est faite de matériau élastomère et a des propriétés conductrices ;
en outre dans laquelle le corps de support (2) a une forme de dôme ayant un côté concave (21a) et un côté convexe (22b), dans laquelle le côté concave (21a) forme le côté contact (21) du corps de support (2), et
dans laquelle
le corps de support (2) est conçu avec une flexibilité telle qu'après l'application de l'électrode sur l'individu, une force exercée centralement sur le côté connecteur (22) et parallèle à l'axe central (A) conduit à une courbure vers le haut de la région radialement extérieure (23) du corps de support (2) en direction du côté connecteur (22),
dans laquelle la courbure vers le haut de la région radialement extérieure (23) du corps de support (2) conduit à une inclinaison de la pluralité de broches de contact extérieures (3) par rapport à l'axe central (A) de telle façon qu'une pointe (31) des broches de contact extérieures (3) se déplace radialement vers l'extérieur le long de la zone d'intérêt de l'individu.

2. Électrode souple selon la revendication 1, **caractérisée en ce qu'**un axe longitudinal (P) de chacune de la pluralité de broches de contact extérieures (3) est parallèle à l'axe central (A).

3. Électrode souple selon la revendication 1 ou 2, **caractérisée en ce que** l'électrode (1) comprend en outre une pluralité de broches de contact intérieures (4) situées sur une région intérieure (24) du corps de support (2) plus proche de l'axe central (A) que la région extérieure (23) du corps de support (2) et étant dimensionnée pour être en contact avec l'individu après que l'inclinaison des broches de contact extérieures (3) s'est produite.

4. Électrode souple selon la revendication 3, **caractérisée en ce que** la pluralité de broches de contact extérieures (3) et la pluralité de broches de contact intérieures (4) ont la même longueur.

5. Électrode souple selon la revendication 3 ou 4, **caractérisée en ce que** la pluralité de broches de contact extérieures (3) et la pluralité de broches de contact intérieures (4) sont agencées et dimensionnées de telle façon que pendant que l'électrode (1) est appliquée sur l'individu, la pluralité de broches de contact extérieures (3) est en contact avec la zone d'intérêt avant la pluralité de broches de contact intérieures (4).

6. Électrode souple selon l'une des revendications précédentes, **caractérisée en ce que** la pluralité de broches de contact extérieures (3) et/ou la pluralité de broches de contact intérieures (4) comprend une partie de base conique et une partie d'extrémité libre cylindrique.

7. Électrode souple selon l'une des revendications précédentes, **caractérisée en ce que** le corps de support (2) est un disque en forme de dôme, de préférence un disque circulaire (25).

8. Électrode souple selon l'une des revendications 1 à 6, **caractérisée en ce que** le corps de support (2) comprend un disque central, de préférence un disque circulaire (26) avec une pluralité de pattes (27) dirigées radialement vers l'extérieur, à un angle par rapport à l'axe central (A) inférieur à 90 degrés, de préférence de 30 à 70 degrés, et définissant la région extérieure (23) du corps de support en forme de dôme (2), dans laquelle la pluralité de broches de contact extérieures (3) est agencée sur l'extrémité libre des pattes (27).

9. Électrode souple selon l'une des revendications précédentes, **caractérisée en ce que** le corps de connecteur (22) du corps de support (2) est doté d'un bouton (28) pour connecter électriquement l'électrode (1) à un circuit électronique.

10. Électrode souple selon l'une des revendications précédentes, **caractérisée en ce que** la pointe (31) de la pluralité de broches de contact extérieures (3) comprend une surface inclinée dirigée vers l'axe central (A) du corps de support (2).

11. Électrode souple selon l'une des revendications précédentes, **caractérisée en ce que** le matériau élastomère de l'électrode (1) est un élastomère thermodurci ou un élastomère thermoplastique.

12. Électrode souple selon l'une des revendications précédentes, **caractérisée en ce que** l'électrode est formée d'un seul bloc.
